Europäisches Patentamt

(19) European Patent Office

Office européen des brevets

(11) Numéro de publication: **0 152 358**

A1

(12) # DEMANDE DE BREVET EUROPEEN

(21) Numéro de dépôt: 85400276.3

(22) Date de dépôt: 15.02.85

(51) Int. Cl.⁴: **C 12 N 15/00**
C 12 N 1/16, C 12 P 21/02
//(C12N1/16, C12R1:865)

(30) Priorité: 16.02.84 FR 8402350

(43) Date de publication de la demande:
21.08.85 Bulletin 85/34

(84) Etats contractants désignés:
AT BE CH DE FR GB IT LI LU NL SE

(71) Demandeur: TRANSGENE S.A.
95 rue Saint-Lazare
F-75009 Paris(FR)

(72) Inventeur: Lemoine, Yves
4, rue de Alisiers
F-67100 Strasbourg(FR)

(72) Inventeur: Sondermeyer, Paul
24 rue des Acacias Ostwald
F-67400 Illkirck(FR)

(72) Inventeur: Loison, Gérard
1 rue de l'Aimant
F-67000 Strasbourg(FR)

(72) Inventeur: Aigle, Michel
151, route de Burkel
F-67400 Illkirch(FR)

(72) Inventeur: Lecocq, Jean-Pierre
6, rue du Champ-du-Feu
F-67116 Reichstett(FR)

(74) Mandataire: Warcoin, Jacques et al,
Cabinet Régimbeau 26, avenue Kléber
F-75116 Paris(FR)

(54) Vecteur d'expression dans les levures de l'interleukine-2 levures transformées et procédé de préparation de l'interleukine-2.

(57) La présente invention concerne un vecteur d'expression dans les levures de la protéine mature de l'Interleukine-2, caractérisé en ce qu'il comporte dans le sens de lecture:
- un promoteur de levure,
- la séquence codant pour la protéine IL-2 mature sous le contrôle du promoteur.
Par fermentation de levures transformées par ces vecteurs on obtient l'interleukine-2.

1

## VECTEUR D'EXPRESSION DANS LES LEVURES DE L'INTERLEUKINE-2, LEVURES TRANSFORMEES ET PROCEDE DE PREPARATION DE L'INTERLEUKINE-2.

La présente invention concerne des vecteurs d'expression de l'interleukine-2 dans les levures et la préparation de cette hormone par fermentation.

L'interleukine-2 (ci-après IL-2) est une hormone immuno modulatrice. Cette hormone provoque la multiplication des lymphocytes impliqués dans les défenses de l'organisme.

L'administration d'IL-2 s'est révélée capable de restaurer les défenses immunitaires de souris dépourvues de thymus, c'est pourquoi IL-2 est potentiellement capable de restaurer les déficits immunitaires liés à certaines maladies, à certains traitements (en particulier radiothérapies) ou bien dans le cas des déficits chroniques.

Chez l'individu sain l'IL-2 pourrait être capable d'augmenter les défenses immunitaires et permettre la prévention de certaines infections.

De façon plus précise l'interleukine-2 appelée précédemment facteur de croissance des cellules T (TCGF) est une lymphokine qui est produite par les cellules T activées par la lectine ou les cellules T activées par antigène. De façon plus précise, son activité biologique comprend la stimulation de la croissance in-vivo à long terme des cellules T activées et l'augmentation de la mitogénèse des thymocites et l'induction de la réactivité des cellules T cytotoxiques.

2

0152358

Comme molécules de régulation, elle présente un rôle clé dans l'étude de la nature moléculaire de la différenciation des cellules T, le mécanisme de fonctionnement des cellules T différenciées de même que des récepteurs d'antigènes des cellules T. Comme l'interféron il a été démontré que l'IL-2 augmente l'activité des cellules tueuses naturelles, ce qui suggère une utilité potentielle dans le traitement de certains néoplastes. En outre, l'effet synergique de l'IL-2 dans l'activité antivirale de IFN-γ a été décrite récemment.

Les propriétés physicochimiques de l'IL-2 ont été bien caractérisées :

la protéine d'IL-2 humaine présente un poids moléculaire de 15 000 avec quelques hétérogénéités dues à différents degrés de sialylation. La protéine présente une remarquable stabilité, elle supporte des températures jusqu'à 80° et garde son activité après traitement avec le SDS.

La présente invention concerne plus particulièrement la préparation d'IL-2 par fermentation de levures en utilisant à titre de souches de fermentation des levures transformées par des vecteurs d'expression plasmidiques particuliers.

Le premier clonage d'IL-2 humain a été rapporté par Taniguchi et al (Nature, 302, 305) suivi par Devon et al (Nucl. Acids Res., 11, 4307) qui décrit également l'expression dans E. coli.

Bien que les bactéries soient des hôtes de choix pour la préparation de protéines par les techniques de recombinaison génétique, on préfère en général utiliser comme cellules hôtes des cellules eucaryotes qui produisent des protéines de structure plus proche des protéines humaines.

C'est pourquoi la présente invention concerne de nouveaux vecteurs d'expression de l'IL-2 humain sous forme mature et non fusionnée dans les levures.

Plus particulièrement la présente invention concerne de nouveaux vecteurs d'expression dans les levures de la protéine mature IL-2 caractérisés en ce qu'ils comportent· dans le sens de lecture :

- un promoteur de levure,

- la séquence codant pour la protéine IL-2 mature sous le contrôle du promoteur.

Parmi les promoteurs utilisables, il faut citer plus particulièrement le promoteur de la phosphoglycératekinase (ci-après dénommée PGK). Il est bien entendu possible d'utiliser d'autres promoteurs connus ou bien d'utiliser des promoteurs totalement ou partiellement synthétiques par exemple la séquence de jonction entre le promoteur de PGK et l'ATG de la séquence codant pour la protéine IL-2 mature peut être la suivante :

ATATAAAACAAGATCTATG

l'ATG ou codon d'initiation étant celui du gène codant pour IL-2 mature.

Dans le cas particulier du gène IL-2 la présence sur le plasmide d'une séquence de terminaison particulière n'est pas nécessaire.

Les vecteurs selon l'invention comporteront également de préférence une origine de réplication dans les levures par exemple l'origine de réplication du plasmide 2 µm de levure ainsi que l'ADN codant pour tout élément, n'agissant qu'en cis, et nécessaire à la réplication du plasmide 2 µ.

En outre il est intéressant de disposer sur un des vecteurs d'un gène codant pour un phénotype assurant une sélection des souches transformées.

Ainsi les vecteurs selon l'invention comporteront par exemple le gène ura3 ou leu2 qui confère le phénotype ura3$^+$ ou leu2$^+$ aux levures transformées. Ce phénotype permet de sélectionner les levures transformées par les vecteurs en transformant, par exemple, des levures ura3$^-$ et en sélectionnant sur milieu sans uracile.

4

0152358

Comme une partie de la synthèse peut être effectuée en utilisant des bactéries hôtes, il peut être intéressant que les vecteurs selon l'invention comportent des éléments bactériens.

Ainsi la présence d'une origine de réplication pour un plasmide permettra la réplication du vecteur dans les cellules bactériennes correspondantes, en particulier dans le cas de E. coli on utilisera une origine de réplication fonctionnelle dans E. coli (ori-eco).

Le vecteur en cause peut également comporter un ou plusieurs gènes de résistance à des antibiotiques s'exprimant dans les bactéries, par exemple l'ampicilline dans le cas de pBR322, mais d'autres gènes de résistance peuvent être utilisés, résistance à la tétracycline ($Tet^r$), ou au chloramphénicol ($Cm^r$).

L'incorporation d'un tel gène marqueur est nécessaire pour la sélection des bactéries contenant les transformants porteurs du plasmide selon l'invention pendant les expériences de clonage.

La présente invention concerne en outre les levures notamment les souches de Saccharomyces cereviseae transformées par les vecteurs selon l'invention par des techniques connues et dont certaines seront rappelées dans les exemples.

Enfin l'invention concerne un procédé de préparation de IL-2 humain dans lequel on cultive sur un milieu de culture des levures transformées comme décrit précédemment et dans lequel on récupère ensuite l'IL-2 formée.

Les milieux de culture mis en oeuvre sont connus de l'homme de métier et devront être adaptés à chaque souche cultivée.

IL-2 peut être séparée par des techniques connues après éclatement des cellules.

Mais il est particulièrement intéressant de mettre à profit l'hydrophobicité d'IL-2 qui fait qu'après éclatement des cellules et centrifugation, IL-2 tend à se rassembler dans le culot de centrifugation duquel elle peut être extraite à l'aide de SDS qui met IL-2 en solution.

La présente invention concerne également un procédé d'isolement de l'IL-2 à partir d'une culture cellulaire, caractérisé en ce que :

- on broie les cellules,

- on centrifuge le broyat,

- on traite le culot de centrifugation par une solution de SDS,

- on extrait l'IL-2 de la phase liquide.

La présente invention comporte, bien entendu, d'autres aspects, notamment certains plasmides qui seront décrits dans les exemples ainsi que leurs mutants et dérivés et de façon générale les procédés de fermentation des levures transformées ainsi que l'IL-2 ainsi obtenu.

D'autres caractéristiques et avantages de l'invention seront mieux compris à la lecture des exemples ci-après et des dessins ci-annexés sur lesquels :

- la figure 1 représente la séquence codant pour IL-2 selon Taniguchi,

- la figure 2 représente la séquence codant pour IL-2 mis en oeuvre dans la présente invention,

- la figure 3 représente l'insertion d'une séquence AccI dans TG26,

- la figure 4 schématise la préparation de TG28,

- la figure 5 représente la stratégie de préparation de pTG832,

- la figure 6 représente la structure de pTG853.

Il convient de remarquer que les différentes séquences de nucléotides figurant dans les dessins doivent être considérées comme faisant explicitement partie de la présente description, ces séquences n'ont pas été reproduites dans le corps du texte afin de ne pas l'alourdir inutilement.

1) Procédés généraux

a) Souches de levures

Saccharomyces cereviseae

segregeants TGYlsp1

TGYlsp4

toutes deux ura3⁻, his 3⁻.

Les souches mentionnées précédemment ont été utilisées parce qu'elles étaient disponibles, mais il est bien entendu qu'il est possible d'utiliser d'autres souches dans la mesure où elles présentent certaines caractéristiques essentielles qui sont rappelées dans le cours de la description détaillée.

b) Préparation des ADN

Des mini-préparations d'ADN de plasmides ou de phages M13 sont effectuées comme cela est décrit par Ish-Horowitz (référence 1) à la seule différence que l'ADN est précipité une seconde fois avec de l'éthanol avant d'être utilisé.

Les maxi-préparations sont effectuées comme cela est décrit dans la publication précédente, avec une purification complémentaire par un gradient de densité CsCl/bromure d'éthidium.

c) Techniques de clonages

Le traitement des ADN avec des enzymes de restriction est effectué, sauf indications contraires, en utilisant les conditions indiquées par le fabricant (New England Biolabs, Bethesda Research Laboratories et Boehringer Mannheim).

Lorsque cela est nécessaire, les phosphates des extrémités 5' sont éliminés en utilisant, soit une phosphatase alcaline bactérienne, soit une phosphatase d'intestin de veau, pendant 30 minutes à 37°C dans le tampon d'enzyme de restriction.

La réparation des extrémités cohésives utilisant la polymérase de Klenow (Boehringer Mannheim) est effectuée à 25°C pendant 15 minutes dans un mélange de 50 mMol. Tris HCl, pH 7,8, 5 mMol. $MgCl_2$, 10 mMol. de β-mercaptoéthanol, 0,4 mMol. de dNTPs avec l'enzyme et 10 à 200 µg/ml d'ADN.

La nucléase $S_1$ (Miles) est utilisée à 2 u/µg d'ADN à 25°C pendant 30 minutes dans un milieu 0,3 Mol. NaCl, 0,03 Mol. NaOAc, pH 4,8, 0,003 Mol. $ZnCl_2$.

Bal31 est utilisée selon le procédé de Panayotatos et al. (référence 2). Les ligations sont effectuées à 15°C (sauf lorsque cela est indiqué différemment) pendant 4 à 24 heures en utilisant de l'ADN ligase $T_4$ (Boehringer Mannheim) avec 100 mMol. de NaCl, 66 mMol. de Tris HCl, pH 7,5, 10 mMol. de $MgCl_2$, 0,5 mMol. de spermidine, 0,2 mMol. de EDTA, 2 mMol. de DTT, 1 mMol. d'ATP, 0,1 mg/ml de BSA et 5 à 50 µg/ml d'ADN.

Pour la ligation d'extrémités cohésives, on utilise environ 30 unités/ml de ligase. Pour la ligation des extrémités franches on utilise environ 100 unités/ml de ligase.

Entre les différentes réactions enzymatiques, des échantillons d'ADN sont extraits avec un mélange phénol/ chloroforme puis précipités à l'éthanol. Lorsque cela est nécessaire, du tARN de E. coli ou de levures est utilisé comme entraîneur. Les adaptateurs moléculaires (Collaborative Research, Bethesda Research Laboratories, New England Biolabs) sont préhybridés et utilisés en excès molaire de 10 à 50 fois pour les extrémités franches d'ADN utilisant les conditions de tampon décrites précédemment avec 100 unités/ml de ligase $T_4$ à 4°C pendant 15 heures. Lorsque l'on utilise des adaptateurs non phosphorylés, les adaptateurs qui n'ont pas réagi sont éliminés directement après ligation par précipitation avec le tétrachlorhydrate de spermine (Hoopes et al. - référence 3).

Lorsque l'on utilise des adaptateurs phosphorylés, le mélange de ligation est tout d'abord extrait avec un mélange phénol/chloroforme puis précipité avec de l'éthanol avant coupure spécifique avec les enzymes de restriction appropriées puis précipitation avec le tétrachlorhydrate de spermine.

Les cellules bactériennes compétentes sont préparées puis transformées avec les plasmides ou transfectées par l'ADN de M13 selon les procédés décrits par Dagert et Ehrlich (référence 4).

Les plasmides de levures sont introduits dans les levures suivant la technique aux sels de lithium de Ito et coll 1983.

EXEMPLE 1 : Préparation de TG28

La synthèse du plasmide pTG853 nécessite d'une part la préparation d'un plasmide vecteur dans les levures, le plasmide pTG832 et la préparation d'un plasmide bactérien sur lequel on a cloné le gène de l'IL-2 : TG28.

Ce dernier plasmide a été obtenu par une stratégie complexe mais qui dans le cadre de la présente invention n'est pas essentiel puisque seul le fragment BglII/PvuII de ce plasmide comportant les séquences codant pour IL-2 présente de l'intérêt.

1)    Isolation du clone de cDNA TG26 codant pour IL-2

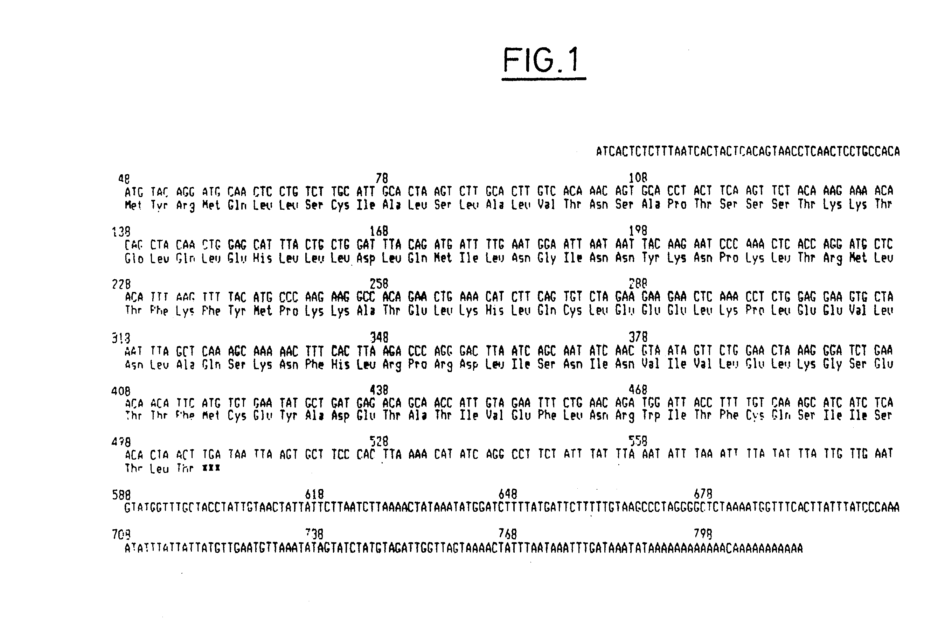

La figure 1 ci-annexée représente la séquence de nucléotides complète codant pour l'IL-2 humaine et qui a été publiée par Taniguchi (Nature, 302, 305). La banque de cDNA utilisée par Taniguchi est obtenue à partir des ARN d'une lignée de cellules T leucémiques JURKAT-3.

Dans le cadre de la présente invention on a mis à profit le fait que IL-2 était également produite par les lymphocytes du sang périphérique ou les lymphocytes de la rate lors d'une induction avec des composés mitogènes. C'est pourquoi le cDNA de l'IL-2 humain a été isolé à partir d'une banque de cDNA de lymphocytes induits par des agents mitogènes.

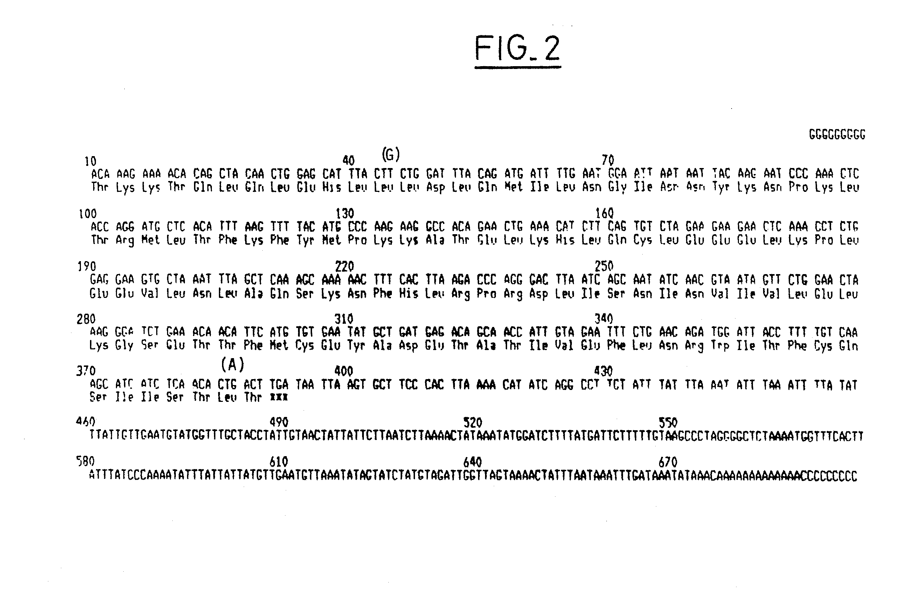

Afin d'effectuer le tri parmi les cDNA de la banque obtenue, on a utilisé une sonde de 19 nucléotides homologues à l'IL-2 humain entre les positions 138 et 157. Cette sonde a été mise en oeuvre selon les techniques classiques utilisées pour sélectionner une colonie bactérienne, d'une banque de cDNA, qui présente un plasmide contenant des insertions avec une taille moyenne de 800 bp. Parmi les 50 000 colonies triées une seule a donné un résultat positif; le plasmide correspondant, dénommé pTG26, a été isolé. La séquence de l'ADN inséré est représentée dans la figure 2..

Une comparaison des séquences données dans les figures 1 et 2 montre certaines différences, ces dernières sont reprises dans la figure 2 (toutefois ces différences ne modifient pas la nature de l'amino-acide codé).

Il convient, en outre, de remarquer que dans le plasmide pTG26 l'extrémité 5' codant pour le peptide signal et les 6 premiers amino-acides de la protéine mature manquent.

2) Remaniement de TG26

Comme cela a été indiqué précédemment il est intéressant de disposer de l'IL-2 sous forme non fusionnée.

Compte tenu de ce qui a été indiqué précédemment, il convient de compléter le clone pTG26 en introduisant en position 5' les séquences codant pour les 7 premiers amino-acides de l'IL-2 humain.

Pour ce faire on synthétise une molécule adaptatrice qui code pour les 7premiers amino-acides F-met-ala-pro-thr-ser-ser-ser de la protéine mature et qui constitue la séquence qui manque dans le clone pTG26.

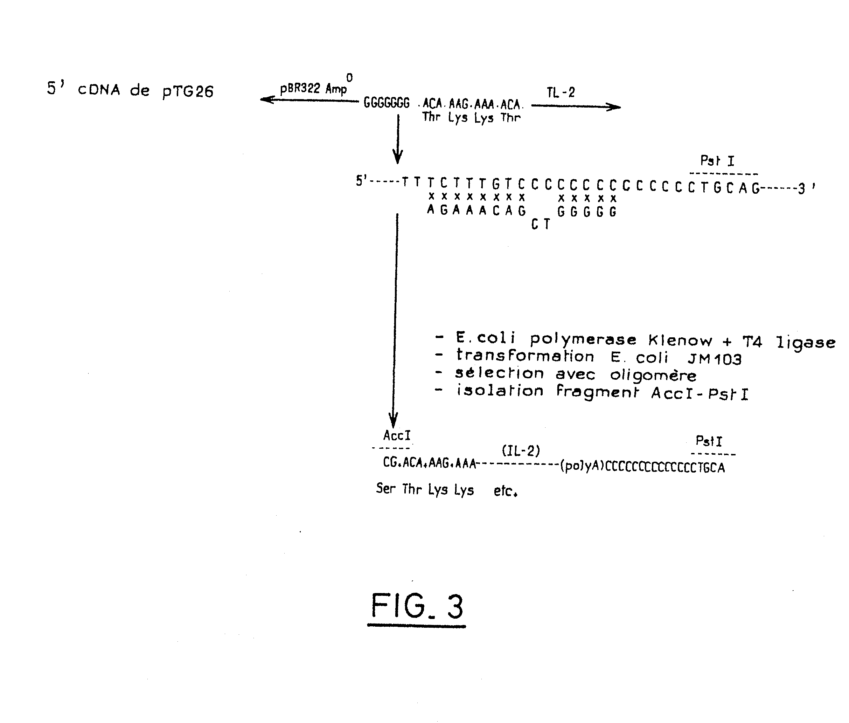

De façon à intégrer cet adaptateur au reste de la séquence codante dans les différents plasmides d'expression, il est nécessaire d'introduire un site de restriction AccI à l'extrémité 5' de l'insert du cDNA de pTG26. Ceci est effectué par mutagénèse in-vitro dirigé par un oligonucléotide en mettant en oeuvre un 15mer utilisant un modèle d'ADN mono-brin provenant d'un dérivé du phage M13 selon le schéma qui est représenté à la figure 3.

On isole ainsi la séquence codant pour l'IL-2 sous forme d'un fragment AccI/PstI auquel manquent les séquences codant pour les 7 premiers amino-acides de la protéine mature.

3) Construction de pTG28

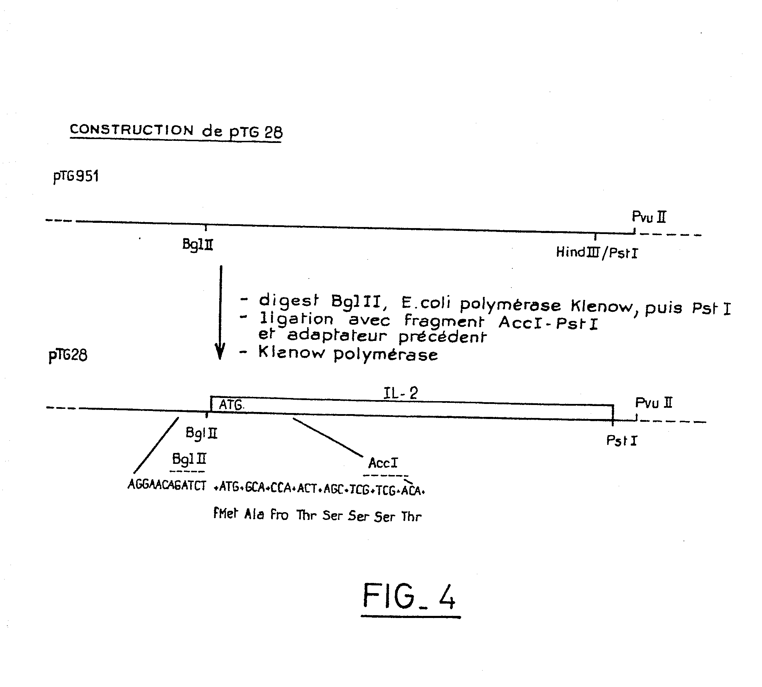

La construction du vecteur pTG28 est représentée à la figure 4.

On part d'un plasmide dénommé pTG951 dont la construction n'est pas essentielle mais qui comporte un site BglII et PstI. Le plasmide pTG951 est mis en digestion avec BglII, les extrémités sont complétées par la polymérase de Klenow, puis l'ensemble est traité avec PstI. Le produit de digestion est mis en ligation avec le fragment AccI/PstI obtenu à partir de pTG26 en utilisant en outre un adaptateur synthétique codant pour les 7 premiers amino-acides. L'adaptateur synthétique est représenté à la figure 4 . La ligation du mélange et le remplissage des trous par la polymérase de Klenow conduisent au plasmide pTG28 qui présente en aval de l'ATG de départ l'ensemble des codons codant pour la protéine IL-2 mature.

La construction de pTG28 a été confirmée par étude de la structure.

EXEMPLE 2 : Vecteur d'expression de l'interleukine-2
dans la levure

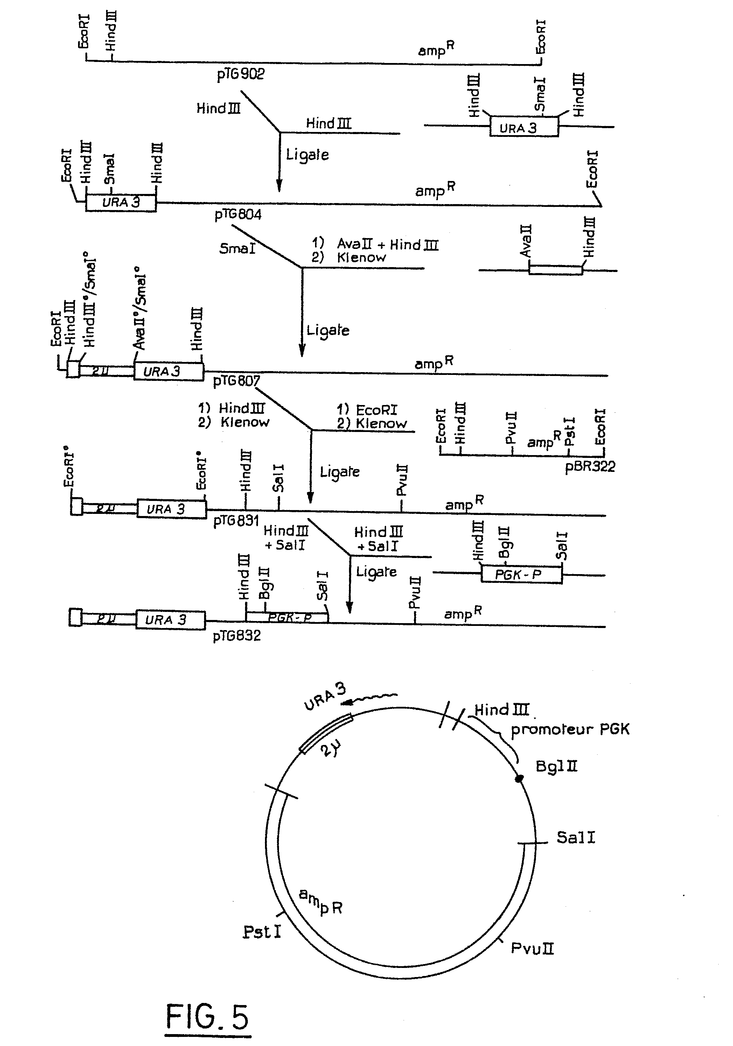

Le vecteur d'expression utilisé est le plasmide pTG832. La construction du plasmide pTG832 est rappelée dans la figure 5.

Le plasmide de départ est le plasmide pTG902 obtenu à partir de pBR322 par délétion des sites PstI et NdeI.

Par digestion de pTG902 et du plasmide portant le gène ura3 (ne possédant pas de site PstI) avec HindIII et ligation du produit de digestion, on obtient le plasmide pTG804.

Le plasmide 2μ (forme B) de levure est mis en digestion avec AvaII et HindIII ; ensuite un traitement à la Klenow permet d'obtenir des extrémités franches. Le morceau AvaII (coordonnée 1295) - HindIII (coordonnée 105) de 1,19 kb est ligé avec pTG803 digéré par SmaI pour fournir le plasmide pTG807.

Le gène ura3 et l'origine de réplication du 2μ sont libérés sous forme d'un fragment unique par digestion de pTG807 avec HindIII.

Ce fragment est traité par la polymérase de Klenow et cloné dans le site EcoRI de pBR322 traité de façon similaire pour donner pTG831.

Le gène de levure phosphoglycerate kinase (PGK) est muté de façon que le site unique BglII soit introduit à l'ATG d'initiation de la traduction de la protéine PGK.

Le fragment HindIII/SalI (2,15 kb), comprenant le promoteur et le début de la région codante du gène correspondant à la phosphoglycérate kinase de levure, a été cloné dans les mêmes sites du vecteur M13mp8.

La création d'un site BglII au niveau de l'ATG initiateur de la phosphoglycératekinase a été effectuée suivant les techniques classiques de mutagénèse in-vitro (Gillam and Smith, 1979 gene 8, 99-106).

Pour ce faire, un oligonucléotide synthétique, de séquence 5' ATATAAAACAAGATCTTTATC 3', a été utilisé afin de modifier la séquence originale 5' ATATAAAACAATGTCTTTATC 3' ; l'ATG initiateur est ainsi rendu non fonctionnel du fait de son remplacement par la séquence AGA.

Le fragment HindIII/SalI (2,15 kb) du gène PGK, ainsi modifié, est cloné dans le plasmide pTG831 pour donner le plasmide pTG832.

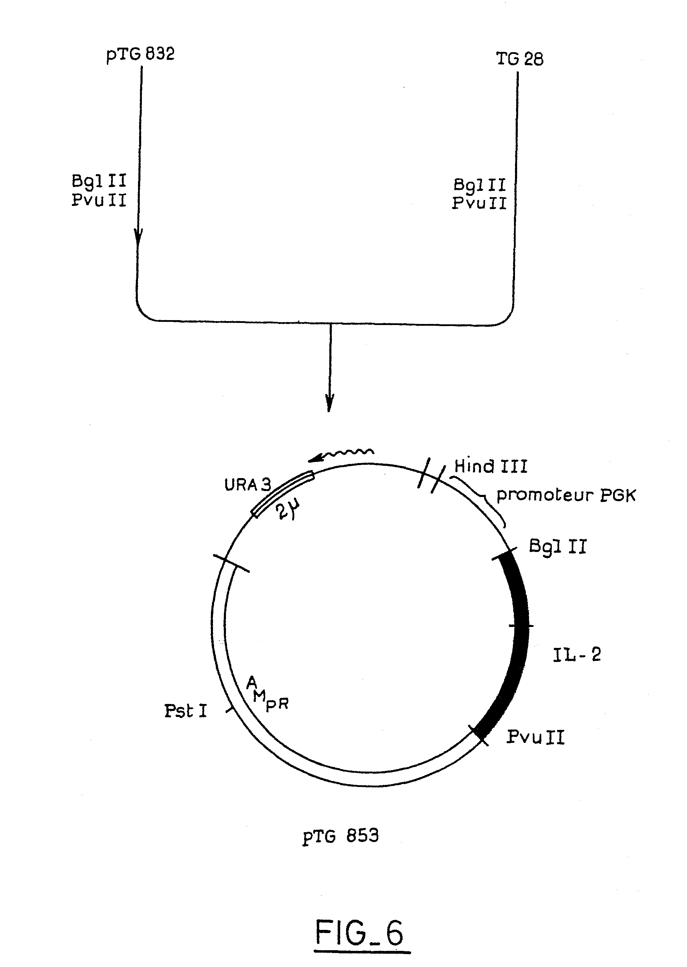

EXEMPLE 3 : Construction du plasmide pTG853 (figure 6)

La partie codante, correspondant à la protéine mature de l'interleukine-2, est introduite sous forme d'un fragment BglII/PvuII (provenant du plasmide pTG951) dans le vecteur pTG832 digéré par les mêmes enzymes de restriction.

La séquence de jonction PGK-IL2 est alors la suivante :
ATATAAAACAAGATCT ATG GCA CCA ACT AGC TCG TCG ACA
                 fmet ala pro thr ser ser ser thr

par comparaison à la séquence de PGK :
ATATAAAACA        ATGTCTTTATC

L'introduction du plasmide pTG853 dans les ségrégeants TGY1sp1 et TGY1sp4 (de génotype ura3⁻, his 3⁻) est effectuée par transformation par acétate de lithium ; la sélection est faite sur milieu minimum synthétique plus histidine (40µg/ml).

EXEMPLE 4 : Dosages de l'activité IL-2

Les ségrégeants TGY1sp1 et TGY1sp4, de génotype ura3⁻, his3⁻, contenant soit le plasmide pTG833 (vecteur d'expression sans le gène de l'IL-2), soit le plasmide pTG853 (vecteur d'expression avec le gène de l'IL-2) sont cultivés sur milieu minimum synthétique (yeast nitrogen base 0,67 %, glucose 2 %) additionné d'histidine (40µg/ml).

Les cellules sont resuspendues dans le tampon PBS (tampon phosphate de K-Na 80mM, NaCl 150 mM) et broyées trois fois une minute par agitation au vortex après addition de billes de verre.

Un extrait est également effectué en présence de SDS 0,1 % ; une dialyse contre du PBS additionné de BSA 1 % (bovin serumalbumine) est effectuée à 4°C pendant trois heures.

Les extraits ainsi obtenus correspondant à 500µl de volume et sont ensuite lyophylisés.

| tampon extraction | ségrégeant | plasmide | unités IL2/ml extract | unités IL2/mg prot. |
|---|---|---|---|---|
| PBS | TGY1sp1 | pTG833 | <0,1 | <0,03 |
| PBS + SDS 0,1% | TGY1sp1 | pTG833 | <0,1 | <0,03 |
| PBS | TGY1sp4 | pTG833 | <0,1 | <0,10 |
| PBS | TGY1sp1 | pTG853 | 7 | 1,84 |
| PBS + SDS 0,1% | TGY1sp1 | pTG853 | 1,9 | 0,5 |
| PBS | TGY1sp4 | pTG853 | 2,5 | 1,60 |

La souche de S. cerevisiae TGY1sp4 contenant le plasmide pTG853 a été déposée le 15 février 1985 sous le numéro I-422 dans la Collection Nationale de Cultures de Microorganismes, 28 rue du Docteur Roux, 75724 Paris Cédex 15.

14

0152358

# Bibliographie

1. Ish-Horowitz D. et Burke J.F., Nucl. Acids Res. 9, 2989-2998 (1981).

2. Panayotatos N. et Trong K., Nucl. Acids Res. 9, 5679-5688 (1981).

3. Hoopes B.C. et McClure R.R., Nucl. Acids Res. 9, 5493-5504 (1981).

4. Dagert M. et Ehrlich S.D., Gene, 23-28 (1979).

5. Shimatake H. et Rosenberg M., Nature, 292, 128-132, (1981).

6. Oppenheimer A.B., Gottesman S. et Gottesman M., J. Mol. Biol., 158, 327-346 (1982).

7. H. Ito, Y. Fukuda, K. Murata, A. Kimura (1983) J. Bacterio 153, 1 163-168.

15    **0152358**

REVENDICATIONS

1. Vecteur d'expression dans les levures de la protéine mature d'IL-2 caractérisé en ce qu'il comporte dans le sens de lecture :
- un promoteur de levure,
- la séquence codant pour la protéine IL-2 mature sous le contrôle du promoteur.

2. Vecteur selon la revendication 1 caractérisé en ce que le promoteur est le promoteur de PGK.

3. Vecteur selon la revendication 2 caractérisé en ce que la séquence de jonction entre le promoteur de PGK et l'ATG de la séquence codant pour la protéine IL-2 mature est la suivante :
ATA TAA AAC AA GATCT A̅T̅G̅.

4. Vecteur selon les revendications 1 à 3 caractérisé en ce qu'il comporte l'origine de réplication d'un plasmide de levure.

5. Vecteur selon la revendication 4 caractérisé en ce qu'il comporte l'origine de réplication du plasmide 2µ ainsi que l'ADN codant pour tout élément, n'agissant qu'en cis, et nécessaire à la réplication du plasmide 2u.

6. Vecteur selon les revendications 1 à 5 caractérisé en ce qu'il comporte la séquence codant pour l'expression du gène ura3.

7. Vecteur selon les revendications 1 à 6 caractérisé en ce qu'il comporte en outre l'origine de réplication d'un plasmide bactérien.

8. Vecteur selon la revendication 7 caractérisé en ce que l'origine de réplication est celle de pBR322.

9. Vecteur selon les revendications 1 à 8 caractérisé en ce qu'il comporte un gène codant pour la résistance à un antibiotique.

0152358

10. Levure transformée par un vecteur selon l'une des revendications 1 à 9.

11. Levure selon la revendication 10 caractérisé en ce qu'il s'agit d'un Saccharomyces cereviseae.

12. Procédé de préparation d'interleukine-2 caractérisé en ce qu'on cultive une levure selon l'une des revendications 10 et 11 sur un milieu de culture et en ce que l'on isole l'inteleukine-2 après culture.

13. Procédé selon la revendication 12, caractérisé en ce qu'à partir d'un milieu de culture et après éclatement des cellules, on centrifuge le broyat cellulaire, on récupère le culot de centrifugation, on traite ce culot par une solution de SDS et on récupère l'IL-2 dans la phase liquide SDS.

14. Interleukine-2 obtenue par la mise en oeuvre du procédé selon l'une des revendications 12 et 13.

# FIG.1

ATCACTCTCTTTAATCACTACTCACAGTAACCTCAACTCCTGCCACA

ATG TAC AGG ATG CAA CTC CTG TCT TGC ATT GCA CTA AGT CTT GCA CTT GTC ACA AAC AGT GCA CCT ACT TCA AGT TCT ACA AAG AAA ACA
Met Tyr Arg Met Gln Leu Leu Ser Cys Ile Ala Leu Ser Leu Ala Leu Val Thr Asn Ser Ala Pro Thr Ser Ser Ser Thr Lys Lys Thr

CAG CTA CAA CTG GAG CAT TTA CTG CTG GAT TTA CAG ATG ATT TTG AAT GGA ATT AAT AAT TAC AAG AAT CCC AAA CTC ACC AGG ATG CTC
Glu Leu Gln Leu Glu His Leu Leu Leu Asp Leu Gln Met Ile Leu Asn Gly Ile Asn Asn Tyr Lys Asn Pro Lys Leu Thr Arg Met Leu

ACA TTT AAG TTT TAC ATG CCC AAG AAG GCC ACA GAA CTG AAA CAT CTT CAG TGT CTA GAA GAA GAA CTC AAA CCT CTG GAG GAA GTG CTA
Thr Phe Lys Phe Tyr Met Pro Lys Lys Ala Thr Glu Leu Lys His Leu Gln Cys Leu Glu Glu Glu Leu Lys Pro Leu Glu Glu Val Leu

AAT TTA GCT CAA AGC AAA AAC TTT CAC TTA AGA CCC AGG GAC TTA ATC AGC AAT ATC AAC GTA ATA GTT CTG GAA CTA AAG GGA TCT GAA
Asn Leu Ala Gln Ser Lys Asn Phe His Leu Arg Pro Arg Asp Leu Ile Ser Asn Ile Asn Val Ile Val Leu Glu Leu Lys Gly Ser Glu

ACA ACA TTC ATG TGT GAA TAT GCT GAT GAG ACA GCA ACC ATT GTA GAA TTT CTG AAC AGA TGG ATT ACC TTT TGT CAA AGC ATC ATC TCA
Thr Thr Phe Met Cys Glu Tyr Ala Asp Glu Thr Ala Thr Ile Val Glu Phe Leu Asn Arg Trp Ile Thr Phe Cys Gln Ser Ile Ile Ser

ACA CTA ACT TGA TAA TTA AGT GCT TCC CAC TTA AAA CAT ATC AGG CCT TCT ATT TAT TTA AAT ATT TAA ATT TTA TAT TTA TTG TTG AAT
Thr Leu Thr ***

GTATGGTTTGCTACCTATTGTAACTATTATTCTTAATCTTAAAACTATAAATATGGATCTTTTATGATTCTTTTTGTAAGCCCTAGGGGCTCTAAAATGGTTTCACTTATTTATCCCAAA

ATATTTATTATTATGTTGAATGTTAAAATATAGTATCTATGTAGATTGGTTAGTAAAACTATTTAATAAATTTGATAAATATAAAAAAAAAAAAACAAAAAAAAAAA

1/6

0152358

# FIG. 2

```
 10                                          40        (G)                                    70
 ACA AAG AAA ACA CAG CTA CAA CTG GAG CAT TTA CTT CTG GAT TTA CAG ATG ATT TTG AAT GGA ATT AAT AAT TAC AAC AAT CCC AAA CTC
 Thr Lys Lys Thr Gln Leu Gln Leu Glu His Leu Leu Leu Asp Leu Gln Met Ile Leu Asn Gly Ile Asn Asn Tyr Lys Asn Pro Lys Leu

100                                         130                                              160
 ACC AGG ATG CTC ACA TTT AAG TTT TAC ATG CCC AAG AAG GCC ACA GAA CTG AAA CAT CTT CAG TGT CTA GAA GAA GAA CTC AAA CCT CTG
 Thr Arg Met Leu Thr Phe Lys Phe Tyr Met Pro Lys Lys Ala Thr Glu Leu Lys His Leu Gln Cys Leu Glu Glu Glu Leu Lys Pro Leu

190                                         220                                              250
 GAG GAA GTG CTA AAT TTA GCT CAA AGC AAA AAC TTT CAC TTA AGA CCC AGG GAC TTA ATC AGC AAT ATC AAC GTA ATA GTT CTG GAA CTA
 Glu Glu Val Leu Asn Leu Ala Gln Ser Lys Asn Phe His Leu Arg Pro Arg Asp Leu Ile Ser Asn Ile Asn Val Ile Val Leu Glu Leu

280                                         310                                              340
 AAG GGA TCT GAA ACA ACA TTC ATG TGT GAA TAT GCT GAT GAG ACA GCA ACC ATT GTA GAA TTT CTG AAC AGA TGG ATT ACC TTT TGT CAA
 Lys Gly Ser Glu Thr Thr Phe Met Cys Glu Tyr Ala Asp Glu Thr Ala Thr Ile Val Glu Phe Leu Asn Arg Trp Ile Thr Phe Cys Gln

370             (A)                         400                                              430
 AGC ATC ATC TCA ACA CTG ACT TGA TAA TTA AGT GCT TCC CAC TTA AAA CAT ATC AGG CCT TCT ATT TAT TTA AAT ATT TAA ATT TTA TAT
 Ser Ile Ile Ser Thr Leu Thr xxx

460                       490                       520                       550
 TTATTGTTGAATGTATGGTTTGCTACCTATTGTAACTATTATTCTTAATCTTAAAACTATAAATATGGATCTTTTATGATTCTTTTTGTAAGCCCTAGGGGCTCTAAAATGGTTTCACTT

580                       610                       640                       670
 ATTTATCCCAAAATATTTATTATTATGTTGAATGTTAAATATAGTATCTATCTAGATTGGTTAGTAAAACTATTTAATAAATTTGATAAATATAAACAAAAAAAAAAAAAAAACCCCCCCC
```

5' cDNA de pTG26

pBR322 Amp⁰ → GGGGGGG .ACA.AAG.AAA.ACA. TL-2 →
Thr Lys Lys Thr

Pst I
5'------TTTCTTTGTCCCCCCCCCCCCCCCCTGCAG------3'
        xxxxxxxx      xxxxx
        AGAAACAG      GGGGG
                CT

- E.coli polymerase Klenow + T4 ligase
- transformation E. coli JM103
- sélection avec oligomère
- isolation fragment AccI-PstI

AccI                        PstI
-----                       -------
CG.ACA.AAG.AAA-----------(IL-2)-------(polyA)CCCCCCCCCCCCCCCTGCA
Ser Thr Lys Lys   etc.

# FIG. 3

CONSTRUCTION de pTG 28

pTG951

pTG28

- digest BglII, E.coli polymérase Klenow, puis PstI
- ligation avec fragment AccI-PstI et adaptateur précédent
- Klenow polymérase

AGGAACAGATCT ·ATG·GCA·CCA·ACT·AGC·TCG·TCG·ACA·

fMet Ala Pro Thr Ser Ser Ser Thr

FIG_4

FIG.5

6/6     0152358

FIG_6

0152358

Numéro de la demande

# Office européen des brevets

# RAPPORT DE RECHERCHE EUROPEENNE

EP 85 40 0276

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl 4) |
|---|---|---|---|
| A | EP-A-0 091 539 (JAPANESE FOUNDATION FOR CANCER RESEARCH) * Revendications 1-30; figure 2 * | 1 | C 12 N 15/00<br>C 12 N 1/16<br>C 12 P 21/02 //<br>(C 12 N 1/16<br>C 12 R 1:865) |
| D,A | NUCLEIC ACIDS RESEARCH, vol. 11, no. 13, juillet 1983, pages 4307-4323, IRL Press Ltd., Oxford, Cambridge, US; R. DEVOS et al.: "Molecular cloning of human interleukin 2 cDNA and its expression in E. coli" * En entier * | 1 | |
| A | EP-A-0 073 635 (KINGSMAN) * Revendications 1-16 * | 1 | |
| A | FR-A-2 469 454 (ANVAR) * Revendications 1-9 * | 1 | DOMAINES TECHNIQUES RECHERCHES (Int. Cl.4)<br><br>C 12 N |

Le présent rapport de recherche a été établi pour toutes les revendications

| Lieu de la recherche<br>LA HAYE | Date d'achevement de la recherche<br>14-05-1985 | Examinateur<br>DELANGHE L.L.M. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82